# EUROPÄISCHE PATENTSCHRIFT

(11) **EP 3 747 405 B1**
(45) Veröffentlichungstag und Bekanntmachung des Hinweises auf die Patenterteilung: **11.08.2021**
(21) Anmeldenummer: 19177809.1
(22) Anmeldetag: 03.06.2019
(51) Int. Cl.: A61F 2/52, D06Q 1/14

(54) **BRUSTPROTHESE MIT BEFLOCKUNG**
BREAST PROSTHESIS WITH FLOCKING
PROTHÈSE MAMMAIRE POURVUE DE FLOCAGE

(43) Veröffentlichungstag der Anmeldung: 09.12.2020
(73) Patentinhaber: Edelweiss Basics GmbH & Co. KG, 83098 Brannenburg (DE)
(72) Erfinder: Weber-Unger, Georg, 6330 Kufstein (AT)
(74) Vertreter: Kador & Partner PartG mbB

(56) Entgegenhaltungen:
- WO-A1-2012/113552
- DE-A1- 2 827 077
- DE-A1- 19 942 996

## Beschreibung

Die Erfindung betrifft eine Brustprothese mit Beflockung, ein Verfahren zur Herstellung einer beflockten Brustprothese, ein Verfahren zur Beflockung einer Brustprothese, und die Verwendung eines zumindest teilweise beflockten Substrats auf einer Außenseite einer Brustprothese, wie in den Ansprüchen definiert.

Im Bereich der Brustprothesen besteht das beständige Bedürfnis Brustprothesen bereitzustellen, die nicht nur in ihrer Form und Gestalt der natürlichen Brust der Trägerin entsprechen, sondern zeitgleich auch einen hohen Tragekomfort für die Trägerin aufweisen.

Eine Brustprothese liegt im Tragezustand mit ihrer Rückseite auf der operierten Brust der Trägerin auf. Ein hoher Tragekomfort einer solchen Brustprothese zeichnet sich nicht nur durch eine angenehme Haptik auf der operierten Brust der Trägerin aus, sondern auch im Tragezustand der Prothese durch verringerte Schweißbildung auf der operierten Brust aus.

Auch eine einfache Reinigung der Rückseite solcher Brustprothesen erhöht die Hygiene und somit den Tragekomfort.

Aus der DE 202016104677 ist eine Brustprothese mit Polsterung bekannt. Die Rückseite der Brustprothese weist ein Florgewebe auf, wobei das Florgewebe einen Flor und ein Grundgewebe aufweist. Bei einem Florgewebe ist der Flor mit seinem Grundgewebe verbunden, und der Flor steht aus diesem Grundgewebe ab. Der Flor dient als Polsterung auf der Rückseite der Brustprothese.

Die WO 2012/113552 A1 offenbart ein künstliches Brustgewebeteil zum Tragen in der Büstenschale eines Büstenschalen aufweisenden Kleidungsstücks, mit einem Körper, der einen weich-elastischen Kern und zwei längs einer eine geschlossene Schleife bildenden Naht fest miteinander verbundene elastische Kunststofffolien aufweist, die eine Hülle bilden, in die der Kern hohlraumfrei eingeschlossen ist, wobei der Körper eine Rückseite aufweist, die mindestens eine Vertiefung hat, über die sich ein Bereich einer textilen Stofflage erstreckt, die längs einer eine geschlossene Schleife bildenden Naht mit dem Körper fest verbunden ist und eine luftdurchlässige Struktur hat, die einen Luftaustausch zwischen der Vertiefung und der äußeren Umgebung des Brustgewebeteils gestattet, dadurch gekennzeichnet, dass die textile Stofflage zumindest in dem über die Vertiefung sich erstreckenden Bereich eine Vielzahl von im Abstand voneinander angeordneten Belüftungslöchern aufweist, zwischen denen die Stofflage luftdurchlässig ist, sodass die Belüftungslöcher einen zusätzlichen Luftaustausch zwischen der Vertiefung und der äußeren Umgebung des Brustgewebeteils gestatten.

Die DE 199 42 996 A1 offenbart ein Verfahren zur Herstellung eines Unterbekleidungsstückes und ein entsprechendes Unterbekleidungsstück. Die Erfindung zeichnet sich dadurch aus, dass auf einem mindestens einlagigen textilen Trägermaterial bereichsweise ein einseitiger Klebstoffauftrag erfolgt, der nachfolgend mit einem Flockmaterial beschichtet wird. Diese Klebstoffbereiche, die entweder punktförmig oder linienförmig aufgebracht sind, haben ausgezeichnete Stütz- und Formungseigenschaften, und werden in beliebigen Formgebungen auf dem Unterbekleidungsstück an den Stellen angebracht, an denen besondere Stütz- und Formungskräfte erforderlich sind.

Darüber hinaus sollte die Brustprothese, insbesondere die Rückseite der Brustprothese, strapazierfähig und abriebbeständig sein. Der Grund hierfür ist, dass vor allem die Rückseite der Prothese aus hygienischen Gründen gereinigt werden muss. Die Reinigung kann beispielsweise durch Waschen, Ausbürsten oder anderweitige mechanische Vorgänge erfolgen. Diese mechanischen Reinigungen sollen aber nicht die Haptik der Rückseite der Brustprothese beeinträchtigen und somit den Tragekomfort der Brustprothese verringern.

Hierbei soll die vorliegende Erfindung Abhilfe schaffen. Es ist eine Aufgabe der Erfindung eine Brustprothese bereitzustellen, welche die oben erwähnten Nachteile vermeidet.

Es ist eine weitere Aufgabe der Erfindung eine Brustprothese bereitzustellen, welche einen hohen Tragekomfort aufweist und zugleich einfach zu reinigen ist.

Es ist insbesondere eine weitere Aufgabe der Erfindung eine Brustprothese bereitzustellen, dessen Rückseite eine angenehme Haptik auf der operierten Brust der Trägerin hat, Schweißbildung auf der operierten Brust der Trägerin vermindert, wenn nicht vollständig verhindert, eine mechanisch strapazierfähige und abriebbeständige Rückseite aufweist und zugleich eine einfache Reinigung der Rückseite ermöglicht. Eine angenehme Haptik zeichnet sich insbesondere durch einen weiche und samtige Haptik der Rückseite aus.

Der Erfindung liegt die Erkenntnis zugrunde, dass die oben erwähnten Aufgaben durch einen Flock bzw. Fasern, welche mittels eines Klebstoffs auf einem Substrat fixiert sind, gelöst werden können.

Gegenstand der Erfindung ist eine Brustprothese mit Beflockung, wobei die Brustprothese einen Körper umfasst, der eine die Form einer weiblichen Brust aufweisende Vorderseite und eine Rückseite aufweist, dadurch gekennzeichnet, dass die Rückseite der Brustprothese ein Substrat aufweist und das Substrat zumindest teilweise einen Flock aufweist, wobei der Flock zueinander parallel angeordnete Fasern umfasst, welche mittels eines Klebstoffs auf dem Substrat fixiert sind, wie in Anspruch 1 definiert.

Ein weiterer Gegentand der Erfindung ist Verfahren zur Herstellung einer beflockten Brustprothese, umfassend die Schritte
a1) Auftragen eines Klebstoffs auf zumindest eines Teils eines Substrats,
a2) Aufbringen und Ausrichten eines Flocks auf den auf dem Substrat aufgetragenen Klebstoff mittels eines elektrostatischen Feldes,
a3) Fixieren des ausgerichteten Flocks um ein zumindest teilweise beflocktes Substrat zu erhalten,
a4) Formen des zumindest teilweise beflockten Substrats zu einer Brustprothese um eine beflockte Brustprothese zu erhalten, oder

Aufbringen des zumindest teilweise beflockten Substrats auf eine Außenseite einer Brustprothese um eine beflockte Brustprothese zu
erhalten, wie in Anspruch 8 definiert.

Ein weiterer Gegenstand der Erfindung ist ein Verfahren zur Beflockung einer Brustprothese, umfassend die Schritte
b1) Bereitstellung einer Brustprothese,
b2) Auftragen eines Klebstoffs auf zumindest eines Teils einer Außenseite der Brustprothese,
b3) Aufbringen und Ausrichten eines Flocks auf den auf die Außenseite der Brustprothese aufgetragenen Klebstoffs mittels eines elektrostatischen Feldes,
b4) Fixieren des ausgerichteten Flocks um eine beflockte Brustprothese zu erhalten, wie in Anspruch 10 definiert.

Ein weiterer Gegenstand der Erfindung ist die Verwendung eines zumindest teilweise beflockten Substrats auf einer Außenseite einer
Brustprothese, wie in Anspruch 13 definiert.

Die erfindungsgemäße Brustprothese mit Beflockung weist zahlreiche Vorteile auf. Die Brustprothese kann beispielsweise in einen Büstenhalter eingesetzt werden und ist im Tragezustand derart angeordnet, dass die beflockte Seite des Substrats auf der operierten Brust der Trägerin zu liegen kommt. Der erfindungsgemäße Flock bedingt mit seiner speziellen Struktur, dass ein gewisser Abstand zwischen der Rückseite der Brustprothese und der Haut der Trägerin entsteht. Ein solcher Abstand bedingt eine verbesserte Belüftung zwischen der Rückseite der Brustprothese und der Haut der Trägerin. Somit wird eine Kühlung erzielt, die die Schweißentwicklung auf der Haut der Trägerin vermindern, wenn nicht sogar vollständig vermeiden kann.

Die erfindungsgemäße Brustprothese weist aufgrund der an ihrer Rückseite angebrachten Beflockung einen hohen Tragekomfort auf. In üblichen Brustprothesen ist der Körper der Prothese in einem Beutel aus Polyurethanfolie eingeschweißt, sodass die Folie auf der Rückseite der Prothese direkt auf der Haut der Trägerin zu liegen kommt. Die direkt auf der Haut aufliegende Folie erlaubt im Regelfall keinen Raum für Belüftung, führt vielmehr zu erhöhter Schweißbildung und wird oftmals als unangenehm auf der Haut empfunden. Der erfindungsgemäße Flock auf der Rückseite der Brustprothese wird hingegen haptisch als angenehm von der Trägerin empfunden. Der erfindungsgemäße Flock sorgt daher nicht nur für ein angenehmes Tragegefühl für die Trägerin, sondern ist zugleich auch einfach zu reinigen. Eine einfache Reinigung ist für die Hygiene unabdingbar, da beispielsweise eine Schweißentwicklung auf der operierten Brust nicht immer vollkommen verhindert werden kann.

Der erfindungsgemäße Flock ist zudem auch strapazierfähig, das heißt er hält den üblichen mechanischen Belastungen beim Waschen und/oder Ausbürsten der Brustprothese stand ohne seine weiche oder samtige Haptik zu verlieren.

Als weiteren Vorteil der vorliegenden Erfindung ist anzusehen, dass die Beflockung des Substrats vor oder nach Verarbeitung des Substrats zu einer Brustprothese erfolgen kann. Das bedeutet, dass einerseits zuerst das Substrat beflockt werden kann, bevor das beflockte Substrat zu einer Brustprothese weiter verarbeitet wird. Alternativ kann das beflockte Substrat auch auf die Rückseite einer Brustprothese aufgebracht werden. Andererseits kann aber auch die bereits fertige Brustprothese als solche nachträglich beflockt werden. Dies ermöglicht einen hohen Grad an Flexibilität hinsichtlich der Herstellung einer solchen Brustprothese.

Die Rückseite der Brustprothese weist ein Substrat auf. Das Substrat ist vorzugsweise flächig oder folienartig ausgebildet und weist zwei einander gegenüberliegende Oberflächen auf. Vorzugsweise weist nur eine Oberfläche des Substrats oder nur ein Teil des Substrats zumindest teilweise einen Flock auf. Es ist einsichtig, dass das Substrat stets derart an oder auf der Rückseite der Brustprothese angeordnet ist, sodass die beflockte Oberfläche des Substrats oder der beflockte Teil des Substrats im Tragezustand der Brustprothese auf der operierten Brust der Trägerin zu liegen kommt.

In einer Ausführungsform weist die eine Oberfläche des Substrats den erfindungsgemäßen Flock auf. Diese beflockte Oberfläche des Substrats ist zur Trägerin der Brustprothese gewandt. Die gegenüberliegende Oberfläche des Substrats ist mit der Rückseite der Brustprothese verbunden. Die gegenüberliegende Oberfläche des Substrats ist vorzugsweise mit der Rückseite der Brustprothese mittels Verkleben, Vernähen, Vernadeln, Verschweißen oder Kombinationen davon verbunden.

In einer anderen Ausführungsform bildet das Substrat selbst zumindest einen Teil der Rückseite der Brustprothese oder das Substrat selbst bildet die gesamte Rückseite der Brustprothese. Die zur Trägerin gewandte Oberfläche des Substrats weist den erfindungsgemäßen Flock auf.

Vorzugsweise umfasst das Substrat eine Folie, mehr bevorzugt besteht das Substrat aus einer Folie.

Vorzugsweise umfasst die Folie eine Kunststofffolie. Die Kunststofffolie umfasst vorzugsweise eine Polyurethanfolie, Polyethylenefolie, Polypropylenefolie, Polyesterfolie, mehr bevorzugt besteht die Kunststofffolie aus einer Polyurethanfolie.

Der Flock umfasst zueinander parallel angeordnete Fasern, mehr bevorzugt besteht der Flock aus zueinander parallel angeordneten Fasern. Die Fasern sind mittels eines Klebstoffs direkt auf dem Substrat fixiert. Wie weiter unten detaillierter beschrieben wird ein Klebstoff auf zumindest einen Teil des Substrats aufgebracht. Der Teil des Substrats ist vorzugsweise eine Oberfläche des Substrats wie oben beschrieben. Der Flock bzw. die Fasern werden während des Auftragens mittels eines elektrostatischen Feldes ausgerichtet. Das elektrostatische Feld bedingt, dass sich die Fasern zueinander parallel ausrichten. Die Fasern werden ausgerichtet auf den Klebstoff aufgetragen. Das Aufbringen und Ausrichten der Fasern sind parallel ablaufende Vorgänge. Durch anschließendes Fixieren des Flocks bzw. der Fasern im Klebstoff wird die zueinander parallel Anordnung der Fasern festgelegt. Das Fixieren ist vorzugsweise ein dauerhaftes Fixieren. Mit dauerhaft ist gemeint, dass die fixierte ausgerichtete Position der Fasern im Klebstoff weder durch Waschen, Bürsten, Reiben oder anderen ähnlichen mechanischen Einwirkungen geändert wird. Mit "zueinander parallel angeordnet" beziehungsweise "zueinander parallele Anordnung" der Fasern ist gemeint, dass die Längsachsen der Fasern parallel verlaufen. Die Längsachse einer Faser ist jene Achse der Faser, die entlang der längsten Ausrichtung der jeweiligen Faser verläuft.

Vorzugsweise sind zumindest 80% aller Fasern, mehr bevorzugt zumindest 90% aller Fasern, mehr bevorzugt zumindest 95% aller Fasern, mehr bevorzugt zumindest 99% aller Fasern, und am meisten bevorzugt 100% aller Fasern, das heißt alle Fasern, parallel zueinander angeordnet sind.

Vorzugsweise sind die Fasern derart auf dem Substrat angeordnet, dass der Winkel zwischen den Längsachsen der Fasern und der Oberfläche des Substrats zwischen 80° und 100°, mehr bevorzugt zwischen 85° und 95°, weiter mehr bevorzugt zwischen 88° und 92°, und am meisten bevorzugt genau 90° beträgt. Im letzteren Falle von 90° stehen die Fasern genau in einem rechten Winkel von der Oberfläche des Substrats ab.

Vorzugsweise umfassen die Fasern Naturfasern, synthetische Fasern oder Mischungen davon, mehr bevorzugt bestehen die Fasern aus Naturfasern, synthetische Fasern oder Mischungen davon.

Vorzugsweise umfassen die Fasern Baumwollfasern, Modalfasern, Polyethylenfasern, Polyamidfasern, Polyesterfasern oder Mischungen davon, mehr bevorzugt bestehen die Fasern aus Baumwollfasern, Modalfasern, Polyethylenfasern, Polyamidfasern, Polyesterfasern oder Mischungen davon.

Durch die Länge und/oder die Dicke der Fasern lässt sich die Haptik des erfindungsgemäßen Flocks steuern. Die Fasern weisen eine Länge vorzugsweise zwischen 0.1 mm und 4.0 mm, mehr bevorzugt zwischen 0.2 mm und 3.0 mm, mehr bevorzugt zwischen 0.3 mm und 2.0 mm auf, und/oder weisen die Fasern eine Dicke vorzugsweise zwischen 0.3 dtex und 40 dtex, mehr bevorzugt zwischen 0.5 dtex und 30 dtex, mehr bevorzugt zwischen 0.7 dtex und 25 dtex, und am meisten bevorzugt zwischen 0.9 dtex und 22 dtex auf. Die Einheit "dtex" ist im Textilbereich bekannt. 1 dtex entspricht 1 Gramm pro 10.000 Meter.

Vorzugsweise haben alle Fasern dieselbe Länge und/oder dieselbe Dicke.

Der Klebstoff kann grundsätzlich jeder geeignete Klebstoff sein, welcher die Fasern an die Oberfläche des Substrats bindet und dauerhaft fixieren kann, wie oben beschrieben. Vorzugsweise umfasst der Klebstoff einen chemisch härtenden Klebstoff, einen physikalisch abbindenden Klebstoff oder Kombinationen davon.

Der chemisch härtende Klebstoff umfasst beispielsweise Acrylat-Klebstoffe wie Cyanacrylate oder Methacrylate, Polyesterklebstoffe, Epoxidklebstoffe, Polyurethanklebstoffe, Silikonklebstoffe, Phenolharzklebstoffe, Polyimidklebstoffe, Polysulfidklebstoffe, silan-modifizierte Polymere oder Mischungen davon. Chemisch härtende Klebstoffe härten durch eine chemische Reaktion, und je nach Klebstoff erfolgt dies beispielsweise durch Polymerisation, Polyaddition oder Polykondensation. Vorzugsweise umfasst der chemisch härtende Klebstoff Silikonklebstoff oder Polyurethanklebstoff, mehr bevorzugt umfasst der chemisch härtende Klebstoff Silikonklebstoff oder besteht daraus.

Der physikalisch abbindende Klebstoff umfasst beispielsweise lösungsmittelhaltige Nassklebstoffe oder Kontaktklebstoffe. Bei dieser Art von Klebstoffen liegt ein Polymer in einem Lösungsmittel gelöst vor. Das Polymer umfasst beispielsweise Polurethan, Polyvinylacetat, Polychloropren, Kautschuk oder Acrylate. Das Lösungsmittel wird nach Art des Polymers gewählt und umfasst beispielsweise organische Lösungsmittel wie Ester oder Ketone, oder auch Wasser als Lösungsmittel.

Wie oben beschrieben weist das Substrat zumindest teilweise einen Flock auf Mit anderen Worten ist das Substrat nur teilweise beflockt. Diese teilweise Beflockung ist vorzugsweise eine ornamentartige Beflockung. Eine teilweise Beflockung ist vorzugsweise auch eine vollflächige Beflockung, wobei jedoch der äußere Randbereich des Substrats von der Beflockung ausgespart wird. Mit anderen Worten weist der äußere Randbereich des Substrats keinen Flock auf. Die Breite des äußeren Randbereichs ist vorzugsweise zwischen 1 mm und 2 cm, mehr bevorzugt zwischen 5 mm und 1.5 cm, und am meisten bevorzugt zwischen 1 cm und 1.3 cm.

Vorzugsweise weist das Substrat vollflächig einen Flock auf. Vollflächig bedeutet, dass die gesamte der Trägerin zugewandte Oberfläche des Substrats einen Flock aufweist.

Vorzugsweise erstreckt sich das zumindest teilweise einen Flock aufweisende Substrat, wie oben beschrieben, oder das vollflächig einen Flock aufweisende Substrat, wie oben beschrieben, über die gesamte Rückseite der Brustprothese.

Vorzugsweise weist die Vorderseite der Brustprothese ebenfalls ein Substrat auf und das Substrat auf der Vorderseite weist zumindest teilweise einen Flock auf, wobei der Flock zueinander parallel angeordnete Fasern umfasst, welche mittels eines Klebstoffs auf dem Substrat der Vorderseite fixiert sind. Vorzugsweise ist das Substrat sowie der Flock auf der Vorderseite aus denselben Materialien wie das Substrat und der Flock auf der Rückseite der Brustprothese.

Die vorliegende Erfindung betrifft ferner ein Verfahren zur Herstellung einer beflockten Brustprothese sowie ein Verfahren zur Beflockung einer Brustprothese, wie in Ansprüchen 8 und 10 definiert. Charakterisierend für beide erfindungsgemäßen Verfahren ist, dass durch das Anlegen eines elektrostatischen Feldes in einer Beflockungsvorrichtung der Flock bzw. die Fasern auf einem mit Klebstoff beschichteten Substrat ausgerichtet und dauerhaft fixiert werden können. Die Ausrichtung erfolgt dabei durch die Feldlinien des elektrostatischen Feldes. Die beiden erfindungsgemäßen Verfahren können daher auch als elektrostatische Beflockungsverfahren bezeichnet werden.

Beflockungsvorrichtungen und Beflockungsverfahren sind im Stand der Technik bekannt.

Eine beispielhafte Beflockungsvorrichtung umfasst einen Applikator, in welchen die Fasern, also der Flock, gegeben werden. Der Applikator besteht aus einer Kammer, beispielsweise einer Kunststoffkammer, in die die Fasern eingefüllt werden. Aus einer Öffnung können die Fasern austreten, die mit einem Kunststoffsieb, welches ein unkontrolliertes Herausfallen der Fasern verhindert, versehen ist. Auf der Gegenseite der Öffnung ist innen in der Kammer eine Metallplatte als Anode angebracht. Die Kathode bildet das Substrat bzw. der auf das Substrat aufgetragene Klebstoff. Der Applikator wird mit der Öffnung zum Substrat positioniert und eine Spannung zwischen Anode und Kathode angelegt. Durch das so erzeugte elektrostatische Feld werden die aus der Öffnung des Applikators austretenden Fasern bis zum Kontakt mit dem auf dem Substrat befindlichen Klebstoff ausgerichtet und schießen ausgerichtet in den Klebstoff hinein. Mit anderen Worten sind das Aufbringen und das Ausrichten der Fasern parallel ablaufende Vorgänge. Durch die parallele Ausrichtung der Fasern im Klebstoff wird die samtartige bis borstige Konsistenz oder Haptik erhalten.

Die beispielhafte Beflockungsvorrichtung wie oben beschrieben oder andere aus dem Stand der Technik bekannte geeignete Beflockungsvorrichtungen können für die erfindungsgemäßen Verfahren verwendet werden.

Beim ersteren Verfahren zur Herstellung einer beflockten Brustprothese wird ein Substrat zunächst beflockt, und das beflockte Substrat wird entweder auf eine Brustprothese aufgebracht oder aus dem beflockten Substrat selbst wird eine Brustprothese geformt. Es wird also lediglich das Substrat in einer Beflockungsvorrichtung beflockt, bevor dieses beflockte Substrat dann weiter verarbeitet wird um eine beflockte Brustprothese zu erhalten.

Alle Ausführungsformen und bevorzugten Ausführungsformen der Brustprothese mit Beflockung wie oben beschrieben, sind auch Ausführungsformen und bevorzugten Ausführungsformen des Verfahrens zur Herstellung einer beflockten Brustprothese, sofern anwendbar.

Vorzugsweise erfolgt das Auftragen eines Klebstoffs auf zumindest eines Teils eines Substrats in Schritt a1) mittels händischen Auftragens, Siebdruck, Aufspritzen, Tauchbad oder Kombinationen davon.

Das Aufbringen und Ausrichten des Flocks sind parallel ablaufende Vorgänge. Das Aufbringen und Ausrichten des Flocks auf den auf dem Substrat aufgetragenen Klebstoff mittels eines elektrostatischen Feldes in Schritt a2) erfolgt vorzugsweise in einer Beflockungsvorrichtung. Die Beflockungsvorrichtung kann beispielsweise eine Vorrichtung wie oben beschrieben sein. Das Einstellen der angelegten Spannung in der Beflockungsvorrichtung hängt von jeweils verwendeten Flock bzw. den verwendeten Fasern ab.

Das Fixieren des ausgerichteten Flocks im Klebstoff im Schritt a3) erfolgt mittels Härtung des Klebstoffs. Die Härtung erfolgt vorzugsweise durch Trocknen. Das Trocken erfolgt vorzugsweise in einer Trockenkammer oder bei Raumtemperatur, mehr bevorzugt in einer Trockenkammer. Die Trocknungs-Temperatur in der Trockenkammer sowie die Trockungszeit hängen vom jeweils verwendeten Klebstoff ab.

Das Formen des zumindest teilweise beflockten Substrats zu einer Brustprothese in Schritt a4) kann auf unterschiedliche Art bewerkstelligt werden und hängt im Wesentlichen von der Art des Substrates ab. Ist das Substrat eine Folie, wie beispielsweise eine Polyurethanfolie, so wird das Substrat vorzugsweise zu einem Beutel verschweißt. In diesen Beutel wird anschließend eine Füllmasse eingebracht und vorzugsweise vernetzt, um eine beflockte Brustprothese zu erhalten. Die Füllmasse ist vorzugsweise eine Silikon-Kautschuk-Masse. Die Herstellung von Silikon-Kautschuk Brustprothesen in einem Folienbeutel ist im Stand der Technik bekannt.

Kann aus Substrat selbst jedoch nicht in eine Brustprothese geformt werden, so wird alternativ das zumindest teilweise beflockte Substrat auf die Außenseite einer bereits vorgefertigten oder kommerziell erhältlichen Brustprothese aufgebracht um eine beflockte Brustprothese zu erhalten. Das Ausbringen des beflockten Substrats auf die Außenseite einer Brustprothese erfolgt vorzugsweise mittels Verklebens, Vernähens, Vernadeln, Verschweißens oder Kombinationen davon. Die Art des Aufbringens hängt von der Art und Beschaffenheit des Substrats ab.

Das Verfahren zur Beflockung einer Brustprothese ist dadurch gekennzeichnet, dass eine bereitgestellte Brustprothese selbst beflockt wird. Es wird also die Brustprothese als solche beflockt, vorzugsweise in einer Beflockungsvorrichtung wie oben beschrieben. Der Vorteil hierbei ist, dass der Flock bzw. die Fasern direkt an eine Außenseite der Brustprothese appliziert werden. Die Außenseite der Brustprothese dient daher als Substrat.

Die bereitgestellte Brustprothese in Schritt b1) kann eine im Stand der Technik bekannte Brustprothese sein oder eine kommerziell erhältliche Brustprothese.

Vorzugsweise ist die Außenseite der Brustprothese die Rückseite der Brustprothese. Vorzugsweise kann die Außenseite sowohl die Vorderseite als auch die Rückseite der Brustprothese sein. In diesem Fall ist sowohl die Vorderseite als auch die Rückseite der Brustprothese von Flock bedeckt.

Das Aufbringen und Ausrichten des Flocks im Schritt b3) sind parallel ablaufende Vorgänge. Schritt b3) erfolgt vorzugsweise in einer Beflockungsvorrichtung. Die Beflockungsvorrichtung kann beispielsweise eine Vorrichtung wie oben beschrieben sein. Das Einstellen der angelegten Spannung der Beflockungsvorrichtung hängt von jeweils verwendeten Flock bzw. den verwendeten Fasern ab.

Alle Ausführungsformen und bevorzugten Ausführungsformen des Verfahrens zur Herstellung einer beflockten Brustprothese wie oben beschrieben, sind auch Ausführungsformen und bevorzugten Ausführungsformen des Verfahrens zur Beflockung einer Brustprothese, sofern anwendbar.

Die Erfindung betrifft auch die Verwendung eines zumindest teilweise beflockten Substrats auf einer Außenseite einer Brustprothese, wie in Anspruch 13 definiert. Die Außenseite der Brustprothese ist vorzugsweise die Rückseite und/oder die Vorderseite der Brustprothese, mehr bevorzugt die Rückseite der Brustprothese. Die erfindungsgemäße Verwendung erhöht den Tragekomfort und Schweißbildung im Tragezustand der Brustprothese wird vermindert oder gar vermeidet.

Alle Ausführungsformen und bevorzugten Ausführungsformen der Brustprothese mit Beflockung wie oben beschrieben, sind auch Ausführungsformen und bevorzugten Ausführungsformen der Verwendung eines zumindest teilweise beflockten Substrats auf einer Außenseite einer Brustprothese.

Weitere Einzelheiten, Merkmale und Vorteile des Gegenstands der Erfindung ergeben sich aus der nachfolgenden Beschreibung der zugehörigen Figuren.

Darin zeigen
Figur 1 eine Seitenansicht einer ersten beispielhaften Ausführungsform der erfindungsgemäßen Brustprothese,
Figur 2 eine Seitenansicht einer zweiten beispielhaften Ausführungsform der erfindungsgemäßen Brustprothese.

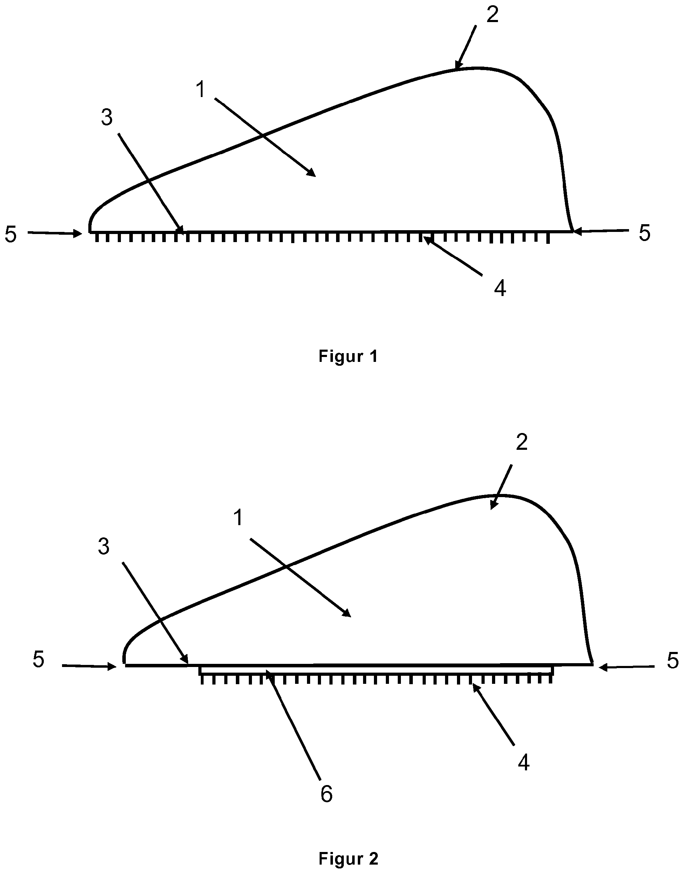

In Figur 1 ist eine Seitenansicht eine erste beispielhafte Ausführungsform der erfindungsgemäßen Brustprothese gezeigt. Die Brustprothese weist einen Prothesenkörper (1) aus einer weich-elastischen Silikon-Kautschukmasse auf, die in einer Polyurethanfolie hohlraumfrei eingeschlossen ist. Der Prothesenkörper weist eine der weiblichen Brust nachgeahmte Vorderseite (2) und eine dem Körper der Trägerin zugewandte Rückseite (3) auf. Der Prothesenkörper (1) hat einen einer geschlossenen Linie folgenden Prothesenrand (5), an dem die Vorderseite (2) und die Rückseite (3) jeweils enden.

Der Flock bzw. die Fasern (4) ist auf dem Bereich der Polyurethanfolie, welcher sich auf der dem Körper der Trägerin zugewandten Rückseite (3) befindet, appliziert worden und bedeckt die Rückseite (3) vollflächig. Die Applikation des Flocks bzw. der Fasern auf die Rückseite (3) der Brustprothese (1) erfolgte mittels des hierin beschriebenen erfindungsgemäßen Verfahrens zur Beflockung einer Brustprothese (1) mit einer oben beschriebenen Beflockungsvorrichtung. Die Polyurethanfolie bildet die Rückseite (3), welche in diesem Beispiel zugleich auch als Substrat (6) dient. Die Fasern sind in diesem Beispiel Polyamidfasern von 0.3 mm Länge und 2 dtex Dicke. Als Klebstoff wurde Silikonkleber verwendet.

Auch Figur 2 zeigt eine beispielhafte Ausführungsform der erfindungsgemäßen Brustprothese. Die Brustprothese weist einen Prothesenkörper (1) aus einer weich-elastischen Silikon-Kautschukmasse auf, die in einer Polyurethanfolie hohlraumfrei eingeschlossen ist. Der Prothesenkörper weist eine der weiblichen Brust nachgeahmte Vorderseite (2) und eine dem Körper der Trägerin zugewandte Rückseite (3) auf. Der Prothesenkörper (1) hat einen einer geschlossenen Linie folgenden Prothesenrand (5), an dem die Vorderseite (2) und die Rückseite (3) jeweils enden. Die Rückseite (3) weist ein beflocktes Substrat (6) auf.

Der Flock bzw. Fasern (4) ist in diesem Ausführungsbeispiel jedoch zuvor auf ein Substrat (6) vollflächig aufgebracht worden. Das Substrat (6) ist hier eine textile Lage aus Baumwolle. Die Applikation des Flocks bzw. der Fasern (4) auf das Substrat (6) erfolgt mittels des hierin beschriebenen erfindungsgemäßen Verfahrens zur Herstellung einer beflockten Brustprothese (1). Erst nach dem Applizieren des Flocks bzw. der Fasern (4) auf eine Oberfläche des Substrats (6) wurde das Substrat (6) mit der Rückseite (3) der Brustprothese (1) verklebt.

Die Fasern sind in diesem Beispiel Polyamidfasern von 0.3 mm Länge und 2 dtex Dicke. Als Klebstoff wurde Silikonkleber verwendet.

### Bezugszeichenliste

- 1: Prothesenkörper
- 2: Vorderseite
- 3: Rückseite
- 4: Flock bzw. Fasern
- 5: Prothesenrand
- 6: Substrat

## Patentansprüche

1. Brustprothese mit Beflockung, wobei die Brustprothese einen Körper (1) umfasst, der eine die Form einer weiblichen Brust aufweisende Vorderseite (2) und eine Rückseite (3) aufweist, wobei die Rückseite (3) der Brustprothese (1) ein Substrat (6) aufweist und das Substrat (6) zumindest teilweise einen Flock (4) aufweist, wobei der Flock (4) zueinander parallel angeordnete Fasern umfasst, welche mittels eines Klebstoffs auf dem Substrat (6) fixiert sind, **dadurch gekennzeichnet, dass** das Substrat (6) eine Folie umfasst.

2. Brustprothese nach einem der vorhergehenden Ansprüche, **dadurch gekennzeichnet, dass** die Fasern Baumwollfasern, Modalfasern, Polyethylenfasern, Polyamidfasern oder Mischungen davon umfassen.

3. Brustprothese nach einem der der vorhergehenden Ansprüche, **dadurch gekennzeichnet, dass** die Fasern eine Länge zwischen 0.1 mm und 4.0 mm aufweisen, und/oder dass die Fasern eine Dicke zwischen 0.3 dtex und 40 dtex mm aufweisen.

4. Brustprothese nach einem der der vorhergehenden Ansprüche, **dadurch gekennzeichnet, dass** die Folie eine Polyurethanfolie umfasst.

5. Brustprothese nach einem der vorhergehenden Ansprüche, **dadurch gekennzeichnet, dass** der Klebstoff einen chemisch härtenden Klebstoff, einen physikalisch abbindenden Klebstoff oder Kombinationen davon umfasst.

6. Brustprothese nach einem der der vorhergehenden Ansprüche, **dadurch gekennzeichnet, dass** das Substrat (6) vollflächig einen Flock (4) aufweist.

7. Brustprothese nach einem der der vorhergehenden Ansprüche, **dadurch gekennzeichnet, dass** die Vorderseite (2) der Brustprothese (1) ebenfalls ein Substrat (6) aufweist und das Substrat (6) auf der Vorderseite (2) zumindest teilweise einen Flock (4) aufweist, wobei der Flock (4) zueinander parallel angeordnete Fasern umfasst, welche mittels eines Klebstoffs auf dem Substrat (6) der Vorderseite (2) fixiert sind.

8. Verfahren zur Herstellung einer beflockten Brustprothese (1), umfassend die Schritte
a1) Auftragen eines Klebstoffs auf zumindest eines Teils eines Substrats (6),
a2) Aufbringen und Ausrichten eines Flocks (4) auf den auf dem Substrat (6) aufgetragenen Klebstoff mittels eines elektrostatischen Feldes,
a3) Fixieren des ausgerichteten Flocks (4) im Klebstoff um ein zumindest teilweise beflocktes Substrat (6) zu erhalten,
a4) Formen des zumindest teilweise beflockten Substrats (6) zu einer Brustprothese um eine beflockte Brustprothese (1) zu erhalten, oder
Aufbringen des zumindest teilweise beflockten Substrats (6) auf eine Außenseite einer Brustprothese (1) um eine beflockte Brustprothese zu erhalten, **dadurch gekennzeichnet, dass** das Substrat (6) eine Folie umfasst.

9. Verfahren nach Anspruch 8, **dadurch gekennzeichnet, dass** das Aufbringen des zumindest teilweise beflockten Substrats (6) auf die Außenseite einer Brustprothese (1) in Schritt a4) mittels Verkleben, Vernähen, Vernadeln, Verschweißen oder Kombinationen davon erfolgt.

10. Verfahren zur Beflockung einer Brustprothese (1), umfassend die Schritte
b1) Bereitstellung einer Brustprothese (1),
b2) Auftragen eines Klebstoffs auf zumindest eines Teils einer Außenseite der Brustprothese (1),
b3) Aufbringen und Ausrichten eines Flocks (4) auf den auf die Außenseite der Brustprothese (1) aufgetragenen Klebstoffs mittels eines elektrostatischen Feldes,
b4) Fixieren des ausgerichteten Flocks (4) im Klebstoff um eine beflockte Brustprothese (1) zu erhalten,
**dadurch gekennzeichnet, dass** die Außenseite eine Rückseite (3) aufweist, wobei die Rückseite (3) ein Substrat aufweist, wobei das Substrat (6) eine Folie umfasst.

11. Verfahren nach einem der Ansprüche 8 bis 10, **dadurch gekennzeichnet, dass** das Fixieren des ausgerichteten Flocks (4) im Klebstoff mittels Härtung des Klebstoffs erfolgt.

12. Verfahren nach Anspruch 11, **dadurch gekennzeichnet, dass** die Härtung durch Trocknen erfolgt.

13. Verwendung eines zumindest teilweise beflockten Substrats (6) auf einer Außenseite einer Brustprothese (1), **dadurch gekennzeichnet, dass** das Substrat (6) eine Folie umfasst.

## Claims

1. Breast prosthesis with flocking, said breast prosthesis comprising a body (1) having a front side (2) in the shape of a female breast and a back side (3), the back side (3) of the breast prosthesis (1) having a substrate (6) and the substrate (6) at least partially having a flock (4), the flock (4) comprising fibres which are arranged parallel to each other and fixed to the substrate (6) by means of an adhesive, **characterised in that** the substrate (6) comprises a film.

2. Breast prosthesis according to one of the preceding claims, **characterised in that** the fibres comprise cotton fibres, modal fibres, polyethylene fibres, polyamide fibres or mixtures thereof.

3. Breast prosthesis according to any one of the preceding claims, **characterised in that** the fibres have a length between 0.1 mm and 4.0 mm, and/or **in that** the fibres have a thickness between 0.3 dtex and 40 dtex mm.

4. Breast prosthesis according to any one of the preceding claims, **characterised in that** the film comprises a polyurethane film.

5. Breast prosthesis according to any one of the preceding claims, **characterised in that** the adhesive comprises a chemically curing adhesive, a physically setting adhesive or combinations thereof.

6. Breast prosthesis according to any one of the preceding claims, **characterised in that** the substrate (6) has a flock (4) over its entire surface.

7. Breast prosthesis according to any one of the preceding claims, **characterised in that** the front side (2) of the breast prosthesis (1) also has a substrate (6) and the substrate (6) on the front side (2) at least partially has a flock (4), the flock (4) comprising fibres arranged parallel to one another which are fixed to the substrate (6) of the front side (2) by means of an adhesive.

8. Method for producing a flocked breast prosthesis (1), comprising the following steps:
a1) applying an adhesive to at least a part of a substrate (6),
a2) applying and aligning a flock (4) to the adhesive applied to the substrate (6) by means of an electrostatic field,
a3) fixing the aligned flock (4) in the adhesive in order to obtain an at least partially flocked substrate (6),
a4) shaping the at least partially flocked substrate (6) into a breast prosthesis in order to obtain a flocked breast prosthesis (1), or
applying the at least partially flocked substrate (6) to an outer side of a breast prosthesis (1) in order to obtain a flocked breast prosthesis, **characterised in that** the substrate (6) comprises a film.

9. Method according to claim 8, **characterised in that** the application of the at least partially flocked substrate (6) to the outside of a breast prosthesis (1) in step a4) is effected by means of gluing, sewing, needling, welding or combinations thereof.

10. Method of flocking a breast prosthesis (1) comprising the following steps:
b1) providing a breast prosthesis (1),
b2) applying an adhesive to at least a part of an outer surface of the breast prosthesis (1),
b3) applying and aligning a flock (4) to the adhesive applied to the outer surface of the breast prosthesis (1) by means of an electrostatic field,
b4) fixing the aligned flock (4) in the adhesive to obtain a flocked breast prosthesis (1),
**characterised in that** the outer side comprises a back side (3), the back side (3) comprising a substrate, the substrate (6) comprising a film.

11. Method according to any one of claims 8 to 10, **characterised in that** the fixing of the aligned flock (4) in the adhesive is effected by means of curing of the adhesive.

12. Method according to claim 11, **characterised in that** the hardening is effected by drying.

13. Use of an at least partially flocked substrate (6) on an outer surface of a breast prosthesis (1), **characterised in that** the substrate (6) comprises a foil.

## Revendications

1. Prothèse mammaire avec flocage, la prothèse mammaire comprenant un corps (1) qui présente une face avant (2) ayant la forme d'un sein de femme et une face arrière (3), la face arrière (3) de la prothèse mammaire (1) présentant un substrat (6) et le substrat (6) présentant au moins partiellement un floc (4), le floc (4) comprenant des fibres disposées parallèlement les unes aux autres, qui sont fixées sur le substrat (6) au moyen d'une colle, **caractérisée en ce que** le substrat (6) comprend une pellicule.

2. Prothèse mammaire selon la revendication précédente, **caractérisée en ce que** les fibres comprennent des fibres de coton, des fibres modales, des fibres de polyéthylène, des fibres de polyamide ou des mélanges de celles-ci.

3. Prothèse mammaire selon l'une quelconque des revendications précédentes, **caractérisée en ce que** les fibres présentent une longueur comprise entre 0,1 mm et 4,0 mm, et/ou **en ce que** les fibres présentent une épaisseur comprise entre 0,3 dtex et 40 dtex.

4. Prothèse mammaire selon l'une quelconque des revendications précédentes, **caractérisée en ce que** la pellicule comprend une pellicule en polyuréthane.

5. Prothèse mammaire selon l'une quelconque des revendications précédentes, **caractérisée en ce que** la colle comprend une colle à durcissement chimique, une colle à prise physique ou des associations de celles-ci.

6. Prothèse mammaire selon l'une quelconque des revendications précédentes, **caractérisée en ce que** le substrat (6) présente un floc (4) sur toute sa surface.

7. Prothèse mammaire selon l'une quelconque des revendications précédentes, **caractérisée en ce que** la face avant (2) de la prothèse mammaire (1) présente également un substrat (6) et le substrat (6) de la face avant (2) présente au moins partiellement un floc (4), le floc (4) comprenant des fibres disposées parallèlement les unes aux autres, qui sont fixées au substrat (6) de la face avant (2) au moyen d'une colle.

8. Procédé pour la fabrication d'une prothèse mammaire (1) floquée, comprenant les étapes :
a1) appliquer une colle sur au moins une partie d'un substrat (6), a2) appliquer et aligner au moyen d'un champ électrostatique un floc (4) sur la colle appliquée sur le substrat (6),
a3) fixer dans la colle le floc (4) aligné pour obtenir un substrat (6) au moins partiellement floqué,
a4) former le substrat (6) au moins partiellement floqué en une prothèse mammaire pour obtenir une prothèse mammaire floquée (1), ou
appliquer le substrat (6) au moins partiellement floqué sur une face extérieure d'une prothèse mammaire (1) pour obtenir une prothèse mammaire floquée, **caractérisé en ce que** le substrat (6) comprend une pellicule.

9. Procédé selon la revendication 8, **caractérisé en ce que** l'application du substrat (6) au moins partiellement floqué sur la face extérieure d'une prothèse mammaire (1) est réalisée à l'étape a4) par collage, couture, aiguilletage, soudage ou des combinaisons de ceux-ci.

10. Procédé pour le flocage d'une prothèse mammaire (1), comprenant les étapes :
b1) fournir une prothèse mammaire (1),
b2) appliquer une colle sur au moins une partie d'une face extérieure de la prothèse mammaire (1),
b3) appliquer et aligner au moyen d'un champ électrostatique un floc (4) sur la colle appliquée sur la face extérieure de la prothèse mammaire (1),
b4) fixer dans la colle le floc (4) aligné pour obtenir une prothèse mammaire (1) floquée,
**caractérisé en ce que** la face extérieure présente une face arrière (3), la face arrière (3) présentant un substrat, le substrat (6) comprenant une pellicule.

11. Procédé selon l'une quelconque des revendications 8 à 10, **caractérisé en ce que** la fixation dans la colle du floc (4) aligné est réalisée par durcissement de la colle.

12. Procédé selon la revendication 11, **caractérisé en ce que** le durcissement est réalisé par séchage.

13. Utilisation d'un substrat (6) au moins partiellement floqué sur une face extérieure d'une prothèse mammaire (1), **caractérisée en ce que** le substrat (6) comprend une pellicule.
